(19) Europäisches Patentamt
European Patent Office
Office européen des brevets

(11) **EP 4 438 239 A1**

(12) **EUROPEAN PATENT APPLICATION**
published in accordance with Art. 153(4) EPC

(43) Date of publication:
**02.10.2024 Bulletin 2024/40**

(21) Application number: **22916760.6**

(22) Date of filing: **28.12.2022**

(51) International Patent Classification (IPC):
*B25J 9/00* (2006.01)          *A61B 5/11* (2006.01)
*A61B 5/00* (2006.01)          *B25J 9/16* (2006.01)
*B25J 13/08* (2006.01)          *B25J 19/02* (2006.01)
*A61H 1/02* (2006.01)

(52) Cooperative Patent Classification (CPC):
**A61B 5/00; A61B 5/11; A61H 1/02; B25J 9/00;**
**B25J 9/16; B25J 13/08; B25J 19/02**

(86) International application number:
**PCT/KR2022/021526**

(87) International publication number:
**WO 2023/128619 (06.07.2023 Gazette 2023/27)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority:  **29.12.2021   KR 20210191239**
               **06.09.2022   KR 20220112702**
               **29.11.2022   KR 20220162486**
               **16.12.2022   KR 20220177413**

(71) Applicant: **Samsung Electronics Co., Ltd.**
**Suwon-si, Gyeonggi-do 16677 (KR)**

(72) Inventor: **SEO, Keehong**
**Suwon-si, Gyeonggi-do 16677 (KR)**

(74) Representative: **Gulde & Partner**
**Patent- und Rechtsanwaltskanzlei mbB**
**Wallstraße 58/59**
**10179 Berlin (DE)**

(54) **METHOD FOR ESTIMATING WALKING INDEX OF USER AND ELECTRONIC DEVICE AND WEARABLE DEVICE FOR PERFORMING SAME**

(57)     Disclosed are a method for estimating a walking index of a user, and an electronic device and a wearable device for performing the same. The electronic device comprises: a communication module for receiving sensor data including movement information of a user wearing the wearable device from the wearable device; a processor for estimating a walking index indicating a walking state of the user on the basis of the sensor data; and a display module for outputting physical ability information including the walking index. The processor estimates the user's walking speed by using a walking speed estimation model which takes the sensor data as an input, estimates the user's step time on the basis of the sensor data, and estimates another walking index on the basis of the walking speed and the step time.

FIG. 8

**Description**

Technical Field

The present disclosure relates to a method of estimating a gait index of a user and an electronic device and wearable device for performing the method.

Background Art

A walking assistance device generally refers to a machine or a device that helps a patient unable to walk on their own because of diseases, accidents, or other causes with their walking exercises for rehabilitation treatment. In our current, rapidly aging society, a growing number of people experience inconvenience when walking or have difficulty with normal walking due to malfunctioning joints, and there is increasing interest in walking assistance devices. A walking assistance device is worn on a user's body to assist the user in walking by providing the necessary muscular strength and to induce the user to walk in a normal walking pattern.

Disclosure of the Invention

Technical Solutions

According to an embodiment, an electronic device includes a communication module configured to receive sensor data including motion information of a user wearing a wearable device from the wearable device, a processor configured to estimate a gait index indicating a walking state of the user, based on the sensor data, and a display module configured to output physical ability information including the gait index. The processor may estimate a walking speed of the user by using a walking speed estimation model with the sensor data as an input. The processor may estimate a step time of the user, based on the sensor data. The processor may estimate another gait index based on the walking speed and the step time.

According to an embodiment, a method of estimating a gait index of a user wearing a wearable device includes receiving sensor data including motion information of the user wearing the wearable device from the wearable device, estimating the gait index indicating a walking state of the user, based on the sensor data, and providing the estimated gait index to the user. The estimating of the gait index may include estimating a walking speed of the user by using a walking speed estimation model with the sensor data as an input. The estimating of the gait index may further include estimating a step time of the user, based on the sensor data. The estimating of the gait index may further include estimating another gait index based on the walking speed and the step time.

Brief Description of Drawings

FIG. 1 is a diagram illustrating an overview of a wearable device worn on a user's body, according to an embodiment.
FIG. 2 is a diagram illustrating a management system including a wearable device and an electronic device, according to an embodiment.
FIG. 3 is a rear schematic diagram illustrating a wearable device according to an embodiment.
FIG. 4 is a left side view illustrating the wearable device according to an embodiment.
FIGS. 5A and 5B are diagrams each illustrating a configuration of a control system of a wearable device, according to an embodiment.
FIG. 6 is a diagram illustrating an interaction between a wearable device and an electronic device, according to an embodiment.
FIG. 7 is a diagram illustrating a configuration of an electronic device according to an embodiment.
FIG. 8 is a flowchart illustrating a method of providing a gait index of a user wearing a wearable device, according to an embodiment.
FIG. 9 is a flowchart illustrating a method of estimating a gait index, according to an embodiment.
FIG. 10 is a diagram illustrating a stride length according to an embodiment.
FIGS. 11A and 11B are diagrams each illustrating an operation of estimating a stride length based on a hip joint angle value, according to an embodiment.
FIG. 12 is a diagram illustrating a gait evaluation result screen based on gait indices, according to an embodiment.
FIG. 13 is a diagram illustrating a configuration and operation of a training device for training a walking speed estimation model, according to an embodiment.

FIG. 14 is a flowchart illustrating operations of a training method of a walking speed estimation model, according to an embodiment.

Best Mode for Carrying Out the Invention

The following detailed structural or functional description is provided as an example only and various alterations and modifications may be made to embodiments. Accordingly, the embodiments are not construed as limited to the disclosure and should be understood to include all changes, equivalents, and replacements within the idea and the technical scope of the disclosure.

As used herein, the singular forms "a", "an", and "the" include the plural forms as well, unless the context clearly indicates otherwise. It will be further understood that the terms "comprises/including" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms, including technical and scientific terms, used herein have the same meaning as commonly understood by one of ordinary skill in the art to which this disclosure pertains. Terms, such as those defined in commonly used dictionaries, are to be interpreted as having a meaning that is consistent with their meaning in the context of the relevant art and are not to be interpreted in an idealized or overly formal sense unless expressly so defined herein.

Hereinafter, embodiments will be described in detail with reference to the accompanying drawings. When describing the embodiments with reference to the accompanying drawings, like reference numerals refer to like elements and a repeated description related thereto will be omitted.

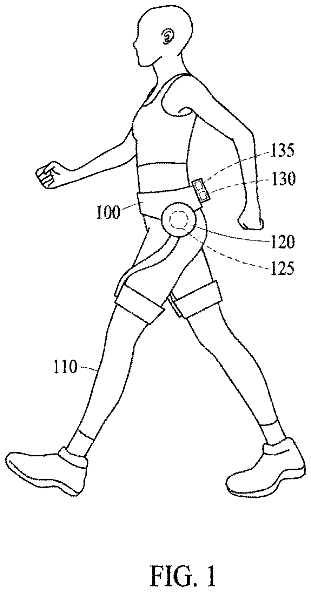

FIG. 1 is a diagram illustrating an overview of a wearable device worn on a user's body, according to an embodiment.

Referring to FIG. 1, a wearable device 100 may be a device worn on a body of a user 110 to assist the user 110 in walking, exercising, and/or working. In an embodiment, the wearable device 100 may be used to measure a physical ability (e.g., a walking ability, an exercise ability, or an exercise posture) of the user 110. In embodiments, the term "wearable device" may be replaced with "wearable robot," "walking assistance device," or "exercise assistance device". The user 110 may be a human or an animal, but examples are not limited thereto. The wearable device 100 may be worn on the body (e.g., a lower body (legs, ankles, knees, etc.) or a waist) of the user 110 and apply an external force, such as an assistance force and/or a resistance force, to a body motion of the user 110. The assistance force may be a force assisting the body motion of the user 110, which is applied in the same direction as a direction of the body motion of the user 110. The resistance force may be a force impeding the body motion of the user 110, which is applied in an opposite direction to the direction of the body motion of the user 110. The term "resistance force" may also be referred to as an "exercise load".

In an embodiment, the wearable device 100 may operate in a walking assistance mode for assisting the walking of the user 110. In the walking assistance mode, the wearable device 100 may assist the walking of the user 110 by applying an assistance force generated through a driving module 120 of the wearable device 100 to the body of the user 110. The wearable device 100 may expand a walking ability of the user 110 by allowing the user 110 to walk independently or walk for a long time by providing a force needed for the walking of the user 110. The wearable device 100 may also improve the walking of a user having an abnormal walking habit or posture.

In an embodiment, the wearable device 100 may operate in an exercise assistance mode to enhance the effect of an exercise on the user 110. In the exercise assistance mode, the wearable device 100 may impede the body motion of the user 110 or resist the body motion of the user 110 by applying a resistance force generated through the driving module 120 of the wearable device 100 to the body of the user 110. When the wearable device 100 is a hip-type wearable device worn on the waist (or pelvis) of the user 110 and the legs (e.g., thighs) of the user 110, the wearable device 100 worn on the legs of the user 110 may enhance the effect of an exercise on the legs of the user 110 by providing an exercise load to the motion of the legs of the user 110. Alternatively, the wearable device 100 may apply an assistance force to the body of the user 110 to assist the exercise of the user 110. For example, when a person with disabilities or an elderly person wears the wearable device 100 to exercise, the wearable device 100 may provide an assistance force to assist body motion in an exercise process. In an embodiment, the wearable device 100 may provide a combination of an assistance force and a resistance force by exercise sessions or time intervals, for example, providing an assistance force in one exercise session and a resistance force in another exercise session.

In an embodiment, the wearable device 100 may operate in a physical ability measurement mode to measure the physical ability of the user 110. The wearable device 100 may measure motion information of a user using sensors (e.g., an angle sensor 125 and an inertial measurement unit (IMU) 135) provided in the wearable device 100 while the user is walking or exercising and may evaluate the physical ability of the user based on the measured motion information. For example, a gait index or an exercise ability indicator (e.g., muscular strength, endurance, balance, or exercise motion) of the user 110 may be estimated through the motion information of the user 110 measured by the wearable device 100. The physical ability measurement mode may include an exercise motion measurement mode to measure an exercise motion of a user.

In embodiments of the present disclosure, for convenience of description, the wearable device 100 is described as an example of a hip-type wearable device, as illustrated in FIG. 1, but the embodiments are not limited thereto. As described above, the wearable device 100 may be worn on another body part (e.g., the upper arms, lower arms, hands, calves, and feet) other than the waist and legs (particularly, the thighs), and the shape and configuration of the wearable device 100 may vary depending on the body part on which the wearable device 100 is worn.

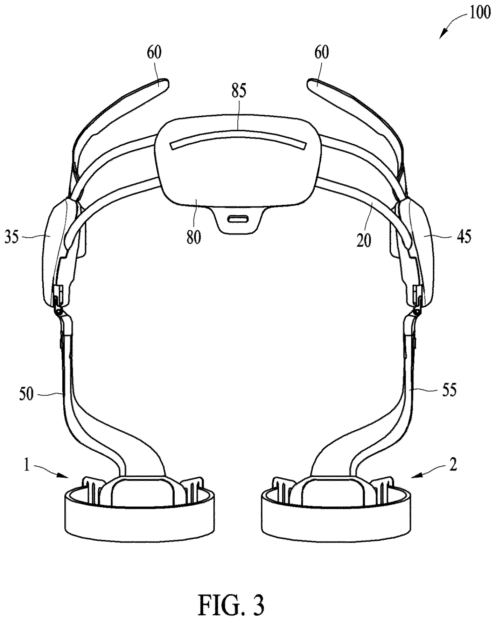

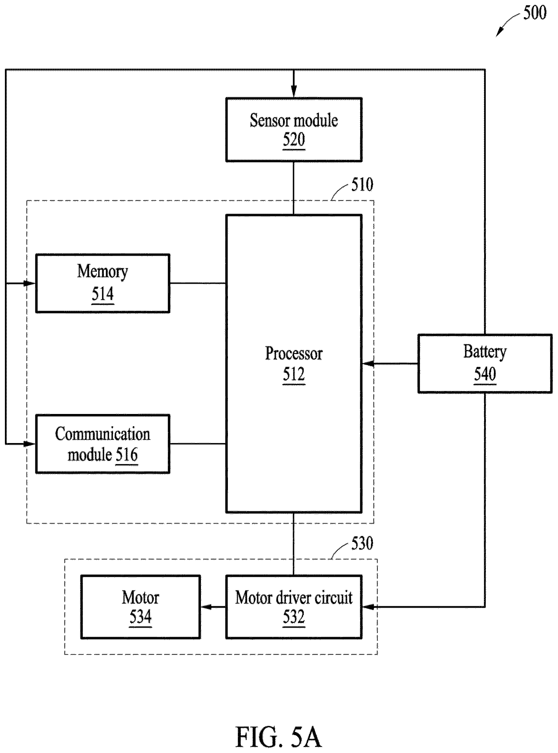

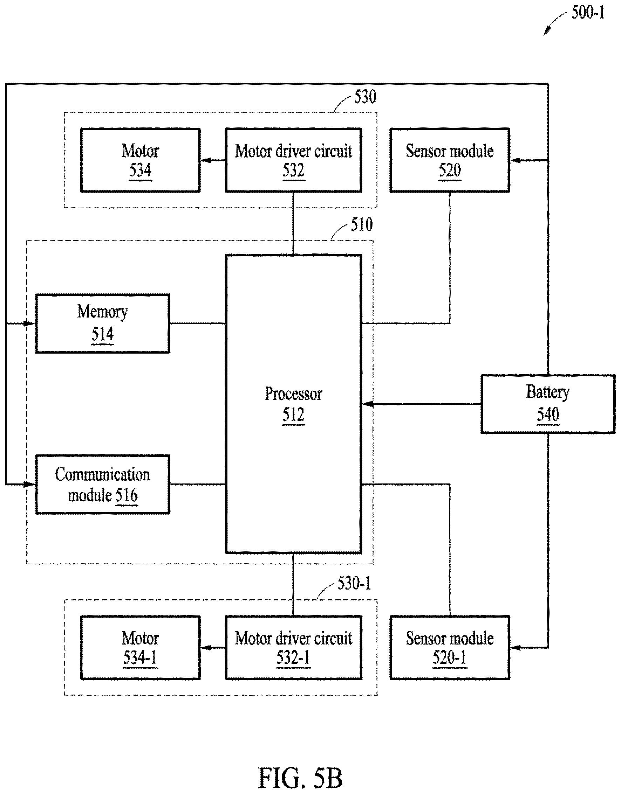

According to an embodiment, the wearable device 100 may include a support frame (e.g., leg support frames 50 and 55 and a waist support frame 20 of FIG. 3) configured to support the body of the user 110 when the wearable device 100 is worn on the body of the user 110, a sensor module (e.g., a sensor module 520 of FIG. 5A) configured to obtain sensor data including motion information about the body motion (e.g., a leg motion and an upper body motion) of the user 110, the driving module 120 (e.g., driving modules 35 and 45 of FIG. 3) configured to generate torque to be applied to the legs of the user 110, and a control module 130 (e.g., a control module 510 of FIGS. 5A and 5B) configured to control the wearable device 100.

The wearable device 100 may include the angle sensor 125 configured to measure the joint angle of the user and the IMU 135 configured to measure a change in acceleration and rotation velocity according to the body motion of the user 110. The angle sensor 125 may measure the rotational angle (or angular velocity) of a leg support frame of the wearable device 100 corresponding to the hip joint angle value of the user 110. The rotational angle of the leg support frame measured by the angle sensor 125 may be estimated as the hip joint angle value (or leg angle value) of the user 110. The angle sensor 125 may include, for example, an encoder and/or a hall sensor. In an embodiment, the angle sensor 125 may be present near each of the right hip joint and the left hip joint of the user 110. The IMU 135 may include an acceleration sensor and/or an angular velocity sensor (e.g., a gyro sensor) and may measure a change in acceleration and/or angular velocity (or rotation velocity) according to the motion of the user 110. The IMU 135 may measure, for example, an upper body motion value of the user 110 corresponding to a motion value of a waist support frame (or a base body (a base body 80 of FIG. 3)) of the wearable device 100. The motion value of the waist support frame measured by the IMU 135 may be estimated as the upper body motion value of the user 110. Herein, the "IMU" may also be referred to as the "inertial sensor".

In an embodiment, the control module 130 and the IMU 135 may be arranged within the base body (e.g., the base body 80 of FIG. 3) of the wearable device 100. The base body may be on the waist (or a lumbar region) of the user 110 when the user 110 wears the wearable device 100. The base body may be formed on or attached to the outside of the waist support frame of the wearable device 100. The base body may be mounted on the lumbar region of the user 110 to provide a cushioning feeling to the lower back of the user 110 and may support the lower back of the user 110 together with the waist support frame.

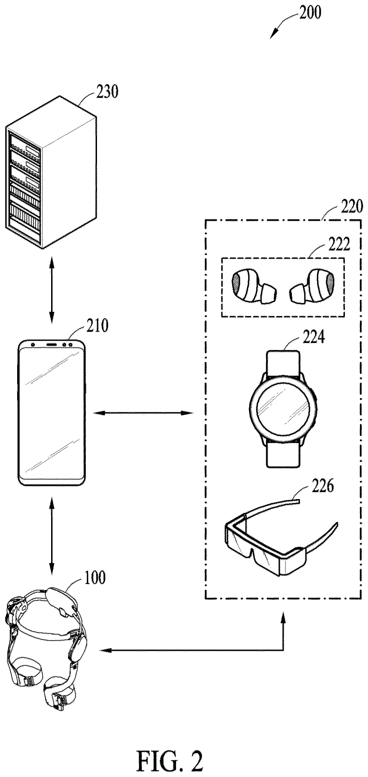

FIG. 2 is a diagram illustrating a management system including a wearable device and an electronic device, according to an embodiment.

Referring to FIG. 2, an exercise management system 200 may include the wearable device 100 to be worn on a body of a user, an electronic device 210, another wearable device 220, and a server 230. In an embodiment, at least one (e.g., the other wearable device 220 or the server 230) of these devices may be omitted from the exercise management system 200, or one or more other devices (e.g., an exclusive controller device of the wearable device 100) may be added to the exercise management system 200.

In an embodiment, the wearable device 100 worn on a body of a user may assist the motion of the user in a walking assistance mode. For example, the wearable device 100 worn on the user's legs may assist the user in walking by generating an assistance force to assist the motion of the user's legs.

In an embodiment, to enhance the effect of an exercise on the user in an exercise assistance mode, the wearable device 100 may generate a resistance force to impede the body motion of the user or an assistance force to assist the body motion of the user and may apply such a force to the body of the user. In the exercise assistance mode, through the electronic device 210, the user may select an exercise program (e.g., squat, split lunge, dumbbell squat, lunge and knee up, stretching, etc.) that the user desires to do for exercise by using the wearable device 100 and/or exercise intensity to be applied to the wearable device 100. The wearable device 100 may control a driving module of the wearable device 100 according to the exercise program selected by the user and may obtain sensor data including motion information of the user through a sensor module. The wearable device 100 may adjust the intensity of a resistance force or an assistance force applied to the user according to the exercise intensity selected by the user. For example, the wearable device 100 may control the driving module to generate a resistive force corresponding to the exercise intensity selected by the user.

In an embodiment, the wearable device 100 may be used to measure a physical ability of the user by interoperating with the electronic device 210. The wearable device 100 may operate in a physical ability measurement mode, which is a mode for measuring the physical ability of the user, by control of the electronic device 210, and may transmit sensor data obtained by the motion of the user in the physical ability measurement mode to the electronic device 210. The electronic device 210 may estimate the physical ability of the user by analyzing the sensor data received from the wearable device 100.

The electronic device 210 may communicate with the wearable device 100 and may remotely control the wearable device 100 or provide the user with state information on a state (e.g., a booting state, a charging state, a sensing state, or an error state) of the wearable device 100. The electronic device 210 may receive the sensor data obtained by a sensor in the wearable device 100 from the wearable device 100 and may estimate the physical ability of the user or an exercise result based on the received sensor data. In an embodiment, when the user exercises while wearing the wearable device 100, the wearable device 100 may obtain sensor data including motion information of the user using sensors and may transmit the obtained sensor data to the electronic device 210. The electronic device 210 may extract a motion value of the user from the sensor data and evaluate an exercise posture of the user based on the extracted motion value. The electronic device 210 may provide the user with an exercise posture measured value and exercise posture evaluation information related to the exercise posture of the user through a graphical user interface (GUI).

In an embodiment, the electronic device 210 may execute a program (e.g., an application) configured to control the wearable device 100, and the user may adjust an operation or a set value of the wearable device 100 (e.g., the magnitude of torque output from a driving module (e.g., the driving modules 35 and 45 of FIG. 3), the volume of audio output from a sound output module (e.g., a sound output module 550 of FIGS. 5A and 5B), or the brightness of a lighting unit (e.g., a lighting unit 85 of FIG. 3) through the corresponding program. The program executed by the electronic device 210 may provide a GUI for interaction with the user. The electronic device 210 may be a device in various forms. For example, the electronic device 210 may include a portable communication device (e.g., a smartphone), a computer device, an access point, a portable multimedia device, or a home appliance (e.g., a television, an audio device, or a projector device), but examples are not limited to the foregoing devices.

In an embodiment, the electronic device 210 may be connected to the server 230 by using short-range wireless communication or cellular communication. The server 230 may receive user profile information of the user of the wearable device 100 from the electronic device 210 and store and manage the received user profile information. The user profile information may include, for example, at least one of a name, an age, a gender, a height, a weight, and a body mass index (BMI). The server 230 may receive exercise history information on an exercise performed by the user from the electronic device 210 and may store and manage the received exercise history information. The server 230 may provide the electronic device 210 with various exercise programs or physical ability measurement programs to be provided to the user.

In an embodiment, the wearable device 100 and/or the electronic device 210 may be connected to the other wearable devices 220. The other wearable devices 220 may include, for example, wireless earphones 222, a smartwatch 224, or smart glasses 226, but examples are not limited to the foregoing devices. In an embodiment, the smartwatch 224 may measure a biosignal including heart rate information of the user and may transmit the measured biosignal to the electronic device 210 and/or the wearable device 100. The electronic device 210 may estimate the heart rate information (e.g., a current heart rate, a maximum heart rate, or an average heart rate) of the user, based on the biosignal received from the smartwatch 224, and may provide the estimated heart rate information to the user.

In an embodiment, the exercise result information, physical ability information (e.g., gait evaluation information), and/or exercise posture evaluation information of the user evaluated by the electronic device 210 may be transmitted to the other wearable device 220 and may be provided to the user through the other wearable device 220. The state information of the wearable device 100 may be transmitted to the other wearable devices 220 and may be provided to the user through the other wearable devices 220. In an embodiment, the wearable device 100, the electronic device 210, and the other wearable devices 220 may be connected to one another through wireless communication (e.g., Bluetooth™ or Wi-Fi communication).

In an embodiment, the wearable device 100 may provide (or output) feedback (e.g., visual, auditory, or tactile feedback) corresponding to the state of the wearable device 100 according to a control signal received from the electronic device 210. For example, the wearable device 100 may provide visual feedback through the lighting unit (e.g., the lighting unit 85 of FIG. 3) and may provide auditory feedback through the sound output module (e.g., the sound output module 550 of FIGS. 5A and 5B). The wearable device 100 may include a haptic module and provide haptic feedback in the form of vibration to the body of the user through the haptic module. The electronic device 210 may also provide (or output) feedback (e.g., visual feedback, auditory feedback, or haptic feedback) corresponding to the state of the wearable device 100.

In an embodiment, the electronic device 210 may present a personalized exercise goal to the user in the exercise assistance mode. The personalized exercise goal may include respective target amounts of exercise for exercise types (e.g., strength exercise, balance exercise, and aerobic exercise) desired by the user, determined by the electronic device 210 and/or the server 230. When the server 230 determines a target amount of exercise, the server 230 may transmit information about the determined target amount of exercise to the electronic device 210. The electronic device 210 may personalize and present the target amounts of exercise for the exercise types, such as strength exercise, aerobic exercise, and balance exercise, according to a desired exercise program (e.g., squat, split lunge, or lunge and knee up) and/or the physical characteristics (e.g., the age, height, weight, and BMI) of the user. The electronic device 210 may display a GUI screen displaying the target amounts of exercise for the respective exercise types on a display.

In an embodiment, the electronic device 210 and/or the server 230 may include a database in which information about a plurality of exercise programs to be provided to the user through the wearable device 100 is stored. To achieve an exercise goal of the user, the electronic device 210 and/or the server 230 may recommend an exercise program suitable for the user. The exercise goal may include, for example, at least one of muscle strength improvement, physical strength improvement, cardiovascular endurance improvement, core stability improvement, flexibility improvement, or symmetry improvement. The electronic device 210 and/or the server 230 may store and manage the exercise program performed by the user, the results of performing the exercise program, and the like.

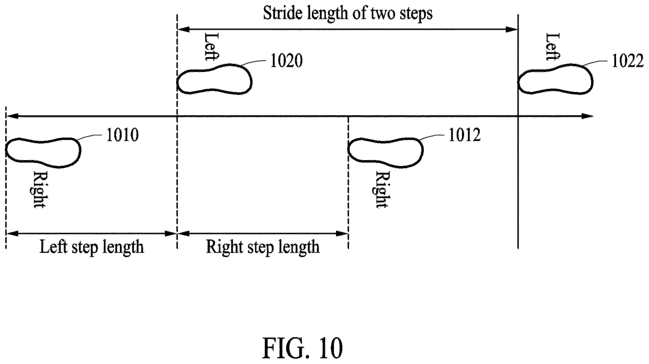

According to an embodiment, the electronic device 210 may evaluate a walking ability of the user by interoperating with the wearable device 100. For example, the electronic device 210 may estimate a gait index, which is an indicator of a walking state of the user, based on sensor data obtained from a sensor module of the wearable device 100 worn by the user. The gait index may be a criterion for determining the quality of walking performed by the user. The gait index estimated by the electronic device 210 may include, for example, at least one of a walking speed, a step time, a step length of one step, a stride length of two steps, a walking distance, a gait symmetry index, a gait variability index, or a walk ratio. The electronic device 210 may calculate the gait index in real time while the user walks wearing the wearable device 100. The step length and the stride length are described in detail with reference to FIG. 10.

When the user walks (or exercises) while wearing the wearable device 100, the wearable device 100 may obtain sensor data including motion information related to the walking of the user using sensors and may transmit the obtained sensor data to the electronic device 210. The electronic device 210 may estimate gait evaluation information of the user, based on the sensor data, and may provide the user with the estimated gait evaluation information. The gait evaluation information may include, for example, various gait indices (e.g., the walking speed, the step time, the stride length, the gait symmetry index, the gait variability index, and the walk ratio) related to the walking of the user when the user walks wearing the wearable device 100. The electronic device 210 may provide the user with feedback information to improve the walking state of the user based on the gait evaluation information. For example, when the measured step/stride length of the user is determined to be less than a desirable step/stride length, the electronic device 210 may propose the user to walk with a wider step/stride length.

The wearable device 100 and the electronic device 210 may estimate the gait index, such as the walking speed, by using an angle sensor (e.g., the angle sensor 125) and an IMU (e.g., the IMU 135) of the wearable device 100, without using a global positioning system (GPS) sensor for tracking the position of the user, and thus, may estimate the gait index not only outdoors but also indoors. In addition, the wearable device 100 and the electronic device 210 may estimate the gait index even when the user walks at a fixed position, such as on a treadmill, and may estimate the gait index reflecting the individual characteristics of the user. The electronic device 210 may provide the user using the wearable device 100 with evaluation information about the walking state of the user to increase the user's interest in walking or exercising.

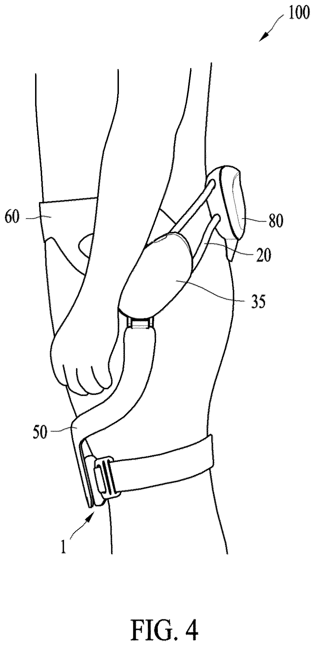

FIG. 3 is a rear schematic diagram illustrating a wearable device according to an embodiment. FIG. 4 is a left side view illustrating the wearable device according to an embodiment.

Referring to FIGS. 3 and 4, the wearable device 100 according to an embodiment may include the base body 80, the waist support frame 20, the driving modules 35 and 45, the leg support frames 50 and 55, thigh fastening portions 1 and 2, and a waist fastening portion 60. The base body 80 may include a lighting unit 85. In an embodiment, at least one (e.g., the lighting unit 85) of these components may be omitted from the wearable device 100, or one or more other components (e.g., a haptic module) may be added to the wearable device 100.

The base body 80 may be on the waist of a user when the user wears the wearable device 100. The base body 80 worn on the waist of the user may cushion and support the waist of the user. The base body 80 may be above a hip of the user when the user wears the wearable device 100 such that the wearable device 100 may not be deviated downward by gravity. The base body 80 may distribute some of a weight of the wearable device 100 to the waist of the user while wearing the wearable device 100. The base body 80 may be connected to the waist support frame 20. Waist support frame connection elements (not shown) which may be connected to the waist support frame 20 may be at both edges of the base body 80.

In an embodiment, the lighting unit 85 may be arranged on the outer side of the base body 80. The lighting unit 85 may include a light source (e.g., light emitting diode (LED)). The lighting unit 85 may emit light by control of a control module (not shown) (e.g., the control module 510 of FIGS. 5A and 5B). In some embodiments, the control module may control the lighting unit 85 to provide (or output) visual feedback corresponding to the state of the wearable device 100 to the user through the lighting unit 85.

The waist support frame 20 may extend from both edges of the base body 80. The waist of the user may be accommodated inside the waist support frame 20. The waist support frame 20 may include one or more rigid body beams. Each beam may be a curved shape with a preset curvature such that the beam may enclose the user's waist.

A waist fastener 60 may be connected to an edge of the waist support frame 20. The driving modules 35 and 45 may be connected to the waist support frame 20.

In an embodiment, the control module, an IMU (not shown) (e.g., the IMU 135 of FIG. 1 or an IMU 522 of FIG. 5B), a communication module (not shown) (e.g., a communication module 516 of FIGS. 5A and 5B), and a battery (not shown) may be arranged inside the base body 80. The base body 80 may protect the control module, the IMU, the communication module, and the battery. The control module may generate a control signal for controlling the operation of the wearable device 100. The control module may include a control circuit including a processor configured to control actuators of the driving modules 35 and 45 and a memory. The control module may further include a power supply module (not shown) to supply power from a battery to each of the components of the wearable device 100.

In an embodiment, the wearable device 100 may include a sensor module (not shown) (e.g., the sensor module 520 of FIG. 5A) for obtaining sensor data from one or more sensors. The sensor module may obtain sensor data that changes according to the motion of a user. In an embodiment, the sensor module may obtain sensor data including motion information of the user and/or motion information of the components of the wearable device 100. The sensor module may include, for example, an IMU (e.g., the IMU 135 of FIG. 1 or the IMU 522 of FIG. 5B) configured to measure an upper body motion value of the user or a motion value of the waist support frame 20 and an angle sensor (e.g., the angle sensor 125 of FIG. 1 or a first angle sensor 520 and a second angle sensor 520-1 of FIG. 5B) configured to measure a hip joint angle value of the user or a motion value of the leg support frames 50 and 55, but is not limited thereto. For example, the sensor module may further include at least one of a position sensor, a temperature sensor, a biosignal sensor, and a proximity sensor.

The waist fastener 60 may be connected to the waist support frame 20 and fix the waist support frame 20 to the waist of the user. The waist fastener 60 may include, for example, a pair of belts.

The driving modules 35 and 45 may generate an external force (or torque) to be applied to the body of the user based on the control signal generated by the control module. For example, the driving modules 35 and 45 may generate an assistance force or resistance force to be applied to the legs of the user. In an embodiment, the driving modules 35 and 45 may include a first driving module 45 in a position corresponding to a position of a right hip joint of the user and a second driving module 35 in a position corresponding to a position of a left hip joint of the user. The first driving module 45 may include a first actuator and a first joint member, and the second driving module 35 may include a second actuator and a second joint member. The first actuator may provide power to be relayed to the first joint member, and the second actuator may provide power to be relayed to the second joint member. The first actuator and the second actuator may each include a motor configured to generate power (or torque) by receiving electric power from the battery. When driven by receiving power, the motors may generate a force (an assistance force) to assist the body motion of the user or a force (a resistance force) to impede the body motion of the user. In an embodiment, the control module may adjust the intensity and direction of the power generated by the motors by adjusting a voltage and/or a current supplied to the motors.

In an embodiment, the first joint member and the second joint member may receive power respectively from the first actuator and the second actuator and may apply an external force to the body of the user, based on the received power. The first joint member and the second joint member may respectively be at positions corresponding to joints of the user. One side of the first joint member may be connected to the first actuator, and the other side of the first joint member may be connected to a first leg support frame 55. The first joint member may rotate by the power relayed from the first actuator. An encoder or a Hall sensor that may operate as an angle sensor configured to measure the rotational angle of the first joint member (corresponding to the joint angle of the user) may be arranged on one side of the first joint member. One side of the second joint member may be connected to the second actuator, and the other side of the second joint member may be connected to a second leg support frame 50. The second joint member may rotate by the power relayed from the second actuator. The encoder or the Hall sensor that may operate as an angle sensor configured to measure the rotational angle of the second joint member may be arranged on one side of the second joint member.

In an embodiment, the first actuator may be in a lateral direction of the first joint member and the second actuator may be in a lateral direction of the second joint member. A rotation axis of the first actuator and a rotation axis of the first joint member may be spaced apart from each other, and a rotation axis of the second actuator and a rotation axis of the second joint member may also be spaced apart from each other. However, examples are not limited to the foregoing examples, an actuator and a joint member may share a rotation axis. In an embodiment, actuators may respectively be spaced apart from joint members. In this case, the driving modules 35 and 45 may further include a power transmission module (not shown) for relaying power from the actuators to the joint members. The power transmission module may be a rotary body, such as a gear, or a longitudinal member, such as a wire, a cable, a string, a spring, a belt, or a chain. However, the scope of embodiments is not limited by a power relay structure and a positional relationship between the actuators and joint members described above.

In an embodiment, the leg support frames 50 and 55 may support a leg (e.g., a thigh) of the user when the wearable device 100 is worn on the leg of the user. For example, the leg support frames 50 and 55 may transmit power (torque) generated by the driving modules 35 and 45 to the thighs of the user, and the power may act as an external force to be applied to the motion of the user's legs. As an edge of each of the leg support frames 50 and 55 is connected to the joint members to rotate and the other edge of each of the leg support frames 50 and 55 is connected to the thigh fastening portions 1 and 2, the leg support frames 50 and 55 may transmit the power generated by the driving modules 35 and 45 to the thighs of the user while supporting the thighs of the user. For example, the leg support frames 50 and 55 may push or pull the thighs of the user. The leg support frames 50 and 55 may extend in a longitudinal direction of the thighs of the user. The leg support frames 50 and 55 may bend to wrap around at least a portion of a circumference of the thighs of the user, respectively. The leg support frames 50 and 55 may include the first leg support frame 55 to support a right leg of the user and the second leg support frame 50 to support a left leg of the user.

The thigh fastening portions 1 and 2 may be connected to the leg support frames 50 and 55 and may fix the leg support frames 50 and 55 to the thighs, respectively. The thigh fastening portions 1 and 2 may include a first thigh fastening portion 2 to fix the first leg support frame 55 to a right thigh of the user and a second thigh fastening portion 1 to fix the second leg support frame 50 to a left thigh of the user.

In an embodiment, the first thigh fastener 2 may include a first cover, a first fastening frame, and a first strap, and the second thigh fastener 1 may include a second cover, a second fastening frame, and a second strap. The first and second covers may apply torque generated by the driving modules 35 and 45 to the user's thighs. The first and second covers may be at a side of the user's thighs to push or pull the user's thighs. The first and second covers may be on a front surface of the user's thighs. The first and second covers may be in a circumferential direction of the user's thighs. The first cover and the second cover may extend to both sides from the other edge of the leg support frames 50 and 55 and may include curved surfaces corresponding to the thighs of the user. An edge of the first and second covers may be connected to a fastening frame and the other edge of the first and second covers may be connected to a strap.

The first fastening frame and the second fastening frame may be arranged, for example, to enclose at least some portions of the circumferences of the thighs of the user and prevent the thighs of the user from being separated from the leg support frames 50 and 55. The first fastening frame may have a fastening structure that connects the first cover to the first strap and the second fastening frame may have a fastening structure that connects the second cover to the second strap.

The first strap may enclose the rest of the circumference of the right thigh of the user, which the first cover and the first fastening frame do not enclose, and the second strap may enclose the rest of the circumference of the left thigh of the user, which the second cover and the second fastening frame do not enclose. The first and second straps may include, for example, an elastic material (e.g., a band).

FIGS. 5A and 5B are diagrams each illustrating a configuration of a control system of a wearable device, according to an embodiment.

Referring to FIG. 5A, a wearable device (e.g., the wearable device 100 of FIGS. 1 and 3) may be controlled by a control system 500. The control system 500 may include the control module 510, the sensor module 520, a driving module 530, and a battery 540. The driving module 530 may include a motor 534 for generating power (e.g., torque) and a motor driver circuit 532 for driving the motor 534. Although one sensor module 510 and the driving module 530 including one motor driver circuit 532 and one motor 534 are illustrated in the example of FIG. 5A, this is just an example. Referring to FIG. 5B, as shown in the illustrated example, a plurality of (e.g., two or more) sensor modules 520 and 520-1, motor driver circuits 532 and 532-1, and motors 534 and 534-1 may be provided. The driving module 530 including the motor driver circuit 532 and the motor 534 may correspond to the first driving module 45 of FIG. 3, and a driving module 530-1 including the motor driver circuit 532-1 and the motor 534-1 may correspond to the second driving module 35 of FIG. 3. The following description of each of the sensor module 520, the motor driver circuit 532, and the motor 534 may also be applied to the sensor module 520-1, the motor driver circuit 532-1, and the motor 534-1 that are illustrated in FIG. 5B.

Referring to FIG. 5A, the sensor module 520 may include at least one sensor. The sensor module 520 may include sensor data including motion information of a user. For example, the sensor module 520 may obtain sensor data that changes according to leg motion of the user. The sensor module 520 may transmit the obtained sensor data to the control module 510. The sensor module 520 may include, for example, an IMU, an angle sensor (e.g., an encoder), a position sensor, a proximity sensor, a biosignal sensor, or a temperature sensor. The IMU may measure acceleration information and posture information while the user walks. For example, the IMU may sense acceleration of each of an X-axis, a Y-axis, and a Z-axis and angular velocity of each of the X-axis, the Y-axis, and the Z-axis according to the gait motion of the user. The angle sensor may measure angle information about a hip joint angle of the user. The angle information measured by the angle sensor may include, for example, a hip joint angle of a right leg, a hip joint angle of a left leg, and information on a motion direction of a leg.

The battery 540 may supply power to each component of the wearable device. The wearable device may convert the power of the battery 540 into power suitable for an operating voltage of each component of the wearable device and supply the converted power to each component.

The driving module 530 may generate an external force to be applied to the user's leg by control of the control module 510. The driving module 530 may be in a position corresponding to a position of a hip joint of the user and may generate torque to be applied to the user's leg, based on a control signal generated by the control module 510. The control module 510 may transmit the control signal to the motor driver circuit 532, and the motor driver circuit 532 may control an operation of the motor 534 by generating a current signal (or a voltage signal) corresponding to the control signal and supplying the current signal to the motor 534. The current signal may not be supplied to the motor 534 according to the control signal. When the current signal is supplied to the motor 534, and the motor is driven, the motor 534 may generate a force to assist leg motion of the user or torque to impede the leg motion of the user.

The control module 510 may control an overall operation of the wearable device and may generate a control signal to control each component (e.g., the driving module 530). The control module 510 may include a processor 512, a memory 514, and a communication module 516.

The processor 512 may execute, for example, software to control at least one other component (e.g., a hardware or software component) of the wearable device connected to the processor 512 and may perform various types of data processing or operations. In an embodiment, as at least a part of data processing or operations, the processor 512 may store instructions or data received from another component (e.g., the communication module 516) in the memory 514, may process the instructions or the data stored in the memory 514, and may store result data after the processing in the memory 514. In an embodiment, the processor 512 may include a main processor (e.g., a central processing unit (CPU) or an application processor (AP)) or an auxiliary processor (e.g., a graphics processing unit (GPU), a neural processing unit (NPU), an image signal processor (ISP), a sensor hub processor, or a communication processor (CP)) that is operable independently of or in conjunction with the main processor. The auxiliary processor may be implemented separately from the main processor or as a part of the main processor.

The memory 514 may store various pieces of data used by at least one component (e.g., the processor 512) of the control module 510. The various pieces of data may include, for example, software, sensor data, input data, or output data for instructions related thereto. The memory 514 may include a volatile memory or a non-volatile memory (e.g., a random-access memory (RAM), a dynamic RAM (DRAM), or a static RAM (SRAM)).

The communication module 516 may support establishing a direct (e.g., wired) communication channel or a wireless communication channel between the control module 510 and another component of the wearable device or an external electronic device (e.g., the electronic device 210 or the other wearable devices 220 of FIG. 2) and performing communication via the established communication channel. For example, the communication module 516 may transmit the sensor data and/or gait evaluation information of the user to the external electronic device. According to an embodiment, the communication module 516 may include one or more CPs that are operable independently of the processor 512 and support direct (e.g., wired) or wireless communication. In an embodiment, the communication module 516 may include a wireless communication module (e.g., a cellular communication module, a short-range wireless communication module, or a global navigation satellite system (GNSS) communication module) and/or a wired communication module. A corresponding one of these communication modules may communicate with the other component of the wearable device and/or the external electronic device via a short-range communication network, such as Bluetooth™, Wi-Fi, an ANT, or infrared data association (IrDA), or a long-range communication network, such as a legacy cellular network, a 5G network, a next-generation communication network, the Internet, or a computer network (e.g., a local area network (LAN) or a wide region network (WAN).

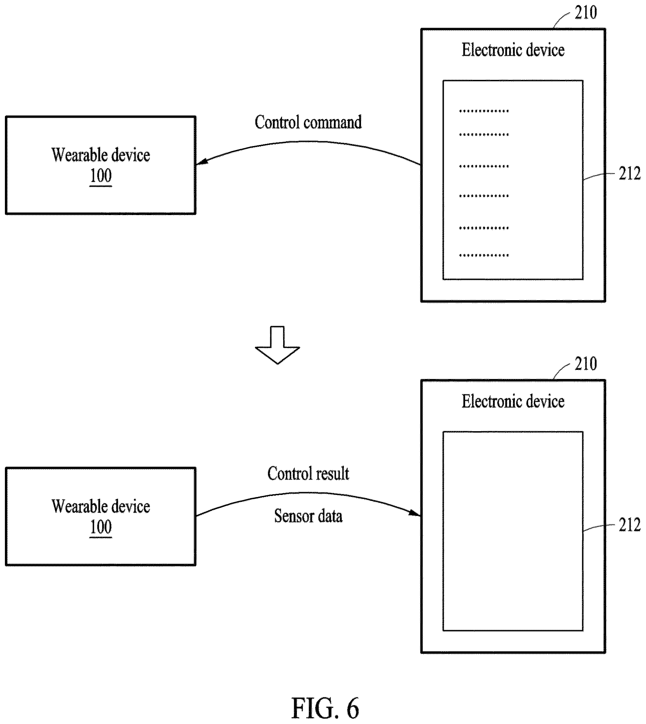

FIG. 6 is a diagram illustrating an interaction between a wearable device and an electronic device, according to an embodiment.

Referring to FIG. 6, a wearable device 100 may communicate with an electronic device 210. For example, the electronic device 210 may be a user terminal of a user using the wearable device 100 or a controller device dedicated to the wearable device 100. In an embodiment, the wearable device 100 and the electronic device 210 may be connected to each other through short-range wireless communication (e.g., Bluetooth™ or Wi-Fi communication).

In an embodiment, the electronic device 210 may check a state of the wearable device 100 or may execute an application to control or operate the wearable device 100. A screen of a user interface (UI) may be displayed to control an operation of the wearable device 100 or determine an operation mode of the wearable device 100 on a display 212 of the electronic device 210 through the execution of the application. The UI may be, for example, a GUI.

In an embodiment, a user may input a command (e.g., a command to execute a walking assistance mode, an exercise assistance mode, or a physical ability measurement mode) to control the operation of the wearable device 100 or may change the setting of the wearable device 100 through a GUI screen on the display 212 of the electronic device 210. The electronic device 210 may generate a control command (or a control signal) corresponding to an operation control command or setting change command input by the user and may transmit the generated control command to the wearable device 100. The wearable device 100 may operate according to the received control command and may transmit a control result according to the received control command and/or sensor data sensed by a sensor module of the wearable device 100 to the electronic device 210. The electronic device 210 may provide the user with result information (e.g., gait ability information, exercise ability information, or exercise motion evaluation information) derived by analyzing the control result and/or the sensor data through the GUI screen.

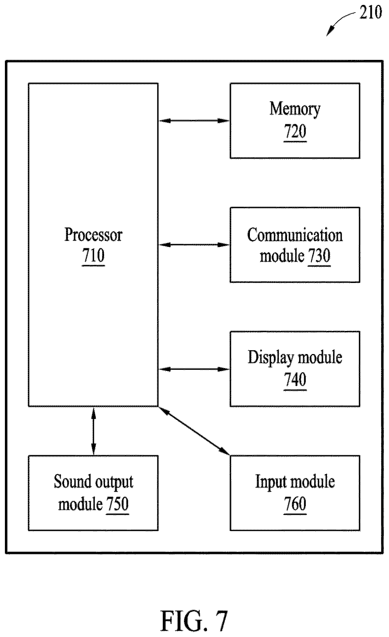

FIG. 7 is a diagram illustrating a configuration of an electronic device according to an embodiment.

Referring to FIG. 7, an electronic device 210 may include a processor 710, a memory 720, a communication module 730, a display module 740, a sound output module 750, and an input module 760. In an embodiment, at least one (e.g., the sound output module 750) of these components may be omitted from the electronic device 210, or one or more other components (e.g., a sensor module and a battery) may be added thereto.

The processor 710 may control at least one other component (e.g., a hardware or software component) of the electronic device 210 and may perform various types of data processing or operations. In an embodiment, as at least a part of data processing or operations, the processor 710 may store instructions or data received from another component (e.g., the communication module 730) in the memory 720, may process the instructions or the data stored in the memory 720, and may store result data in the memory 720.

In an embodiment, the processor 710 may include a main processor (e.g., a CPU or an AP) or an auxiliary processor (e.g., a GPU, an NPU, an ISP, a sensor hub processor, or a CP) that is operable independently of or in conjunction with the main processor.

The memory 720 may store various pieces of data used by at least one component (e.g., the processor 710 or the communication module 730) of the electronic device 210. The various pieces of data may include, for example, a program (e.g., an application) and input data or output data for a command related thereto. The memory 720 may include at least one instruction executable by the processor 710. The memory 720 may include a volatile memory or a non-volatile memory.

The communication module 730 may support establishing a direct (e.g., wired) communication channel or a wireless communication channel between the electronic device 210 and another electronic device (e.g., the wearable device 100, the other wearable devices 220, and the server 230) and performing communication via the established communication channel. The communication module 730 may include a communication circuit for performing a communication function. The communication module 730 may include one or more CPs that are operable independently of the processor 710 (e.g., an AP) and support direct (e.g., wired) or wireless communication. In an embodiment, the communication module 730 may include a wireless communication module (e.g., a Bluetooth™ communication module, a cellular communication module, a Wi-Fi communication module, or a GNSS communication module) that performs wireless communication or a wired communication module (e.g., a LAN communication module or a power line communication (PLC) module). For example, the communication module 730 may transmit a control command to the wearable device 100 and may receive, from the wearable device 100, at least one of sensor data including body motion information of a user wearing the wearable device 100, state data of the wearable device 100, and control result data corresponding to the control command.

The display module 740 may visually provide information to the outside (e.g., the user) of the electronic device 210. The display module 740 may include, for example, a light-emitting diode (LCD) or organic light-emitting diode (OLED) display, a hologram device, or a projector device. The display module 740 may further include a control circuit to control the driving of the display. In an embodiment, the display module 740 may further include a touch sensor set to sense a touch or a pressure sensor set to sense the intensity of a force generated by the touch.

The sound output module 750 may output a sound signal to the outside of the electronic device 210. The sound output module 750 may include a guide sound signal (e.g., a driving start sound or an operation error notification sound) based on a state of the wearable device 100 and a speaker for playing musical content or a guide voice. When it is determined that the wearable device 100 is not properly worn on the body of the user, the sound output module 750 may output a guide voice to inform the user that they are wearing the wearable device 100 abnormally or to guide the user to wear the wearable device 100 normally. The sound output module 750 may output, for example, a guide voice corresponding to exercise evaluation information or exercise result information obtained by evaluating an exercise of the user.

The input module 760 may receive a command or data to be used by another component (e.g., the processor 710) of the electronic device 210 from the outside (e.g., the user) of the electronic device 210. The input module 760 may include an input component circuit and receive a user input. The input module 760 may include, for example, a key (e.g., a button) or a touch screen.

According to an embodiment, the electronic device 210 may include the communication module 730 configured to receive the sensor data including the motion information of the user wearing the wearable device 100 from the wearable device 100, the processor 710 configured to estimate a gait index indicating a walking state of the user, based on the sensor data, and the display module 740 configured to output physical ability information including the gait index.

In an embodiment, the processor 710 may estimate the gait index indicating the walking state of the user, based on the sensor data (e.g., the IMU of the wearable device 100 and/or the sensor data obtained by an angle sensor) received from the wearable device 100. The processor 710 may determine an average walking speed in a certain time interval based on an estimated walking speed. The processor 710 may estimate the walking speed of the user by using a walking speed estimation model with the sensor data as an input. The processor 710 may estimate the walking speed of the user in real time through the walking speed estimation model. In an embodiment, angular velocity (rotation velocity) data (e.g., angular velocity data of each of an x-axis, a y-axis, and a z-axis) and acceleration data (e.g., acceleration data of each of the x-axis, the y-axis, and the z-axis) obtained by the IMU of the wearable device 100 may be input to the walking speed estimation model, and the walking speed estimation model may output a walking speed estimation value based on the input angular velocity data and the input acceleration data. In an embodiment, hip-joint angular velocity data (e.g., angular velocity data corresponding to a right hip joint and angular velocity data corresponding to a left hip joint) obtained by the angle sensor of the wearable device 100 together with the angular velocity (rotation velocity) data and the acceleration data obtained by the IMU of the wearable device 100 may be input to the walking speed estimation model, and the walking speed estimation model may output the walking speed estimation value based on the input angular velocity data and acceleration data of the IMU and the input angular velocity data of the angle sensor. In an embodiment, rotational angle data (e.g., a joint angle of the right hip joint and a joint angle of the left hip joint) and the hip-joint angular velocity data obtained by the angle sensor of the wearable device 100 together with the angular velocity (rotation velocity) data and the acceleration data obtained by the IMU of the wearable device 100 may be input to the walking speed estimation model, and the walking speed estimation model may output the walking speed estimation value based on the input angular velocity data and acceleration data of the IMU and the input rotational angle data and angular velocity data of the angle sensor. In an embodiment, the rotational angle data (e.g., the joint angle of the right hip joint and the joint angle of the left hip joint) and the hip-joint angular velocity data obtained by the angle sensor of the wearable device 100 may be input to the walking speed estimation model, and the walking speed estimation model may output the walking speed estimation value based on the input rotational angle data and angular velocity data of the angle sensor.

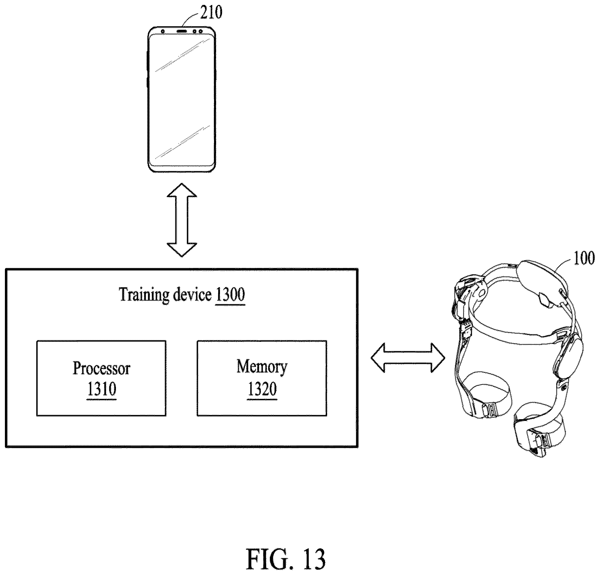

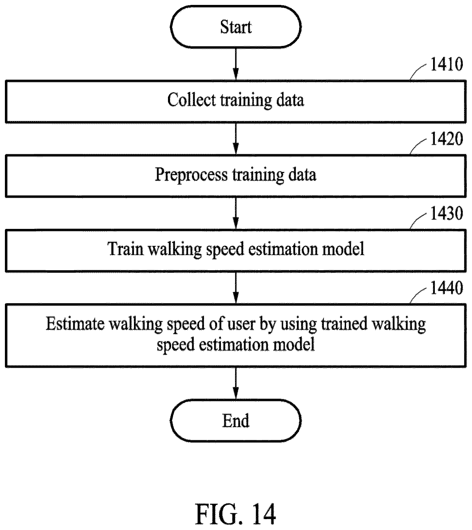

The walking speed estimation model may be a model trained to output a walking speed estimation value corresponding to input sensor data with the sensor data (e.g., the sensor data obtained by the IMU and/or the sensor data obtained by the angle sensor) as an input. For example, the walking speed estimation model may be a model trained through machine learning or linear regression, based on training data (e.g., sensor data measured during walking and an actual walking speed value corresponding to the sensor data). In an embodiment, the walking speed estimation model may be, for example, a convolutional neural network (CNN), a recurrent neural network (RNN), a gated recurrent unit (GRU), a ResNet, a long, short-term memory (LSTM), or a linear regression model, or any combination (e.g., a combination of a CNN and an LSTM) of two or more thereof. The walking speed estimation model may be trained based on a machine learning method of supervised learning. In a training process, an error backpropagation algorithm or a gradient descent algorithm may be used. For example, in the training process, determining and adjusting may be performed repeatedly, in which the determining of a loss is based on a difference between an actual walking speed value and a walking speed estimation value that is output by the walking speed estimation model to which sensor data is input, and the adjusting of parameters (e.g., a connection weight or a bias) of the walking speed estimation model is performed to decrease the loss. Through this training process, an optimal value of hyperparameters that determine the form of the walking speed estimation model may be explored. The training of the walking speed estimation model is described below with reference to FIGS. 13 and 14.

In an embodiment, the processor 710 may estimate a step time of the user based on the sensor data. The step time may be a time spent for the user to take one step, and the processor 710 may measure the step time of an individual step when the user walks while wearing the wearable device. For example, the processor 710 may detect a start of a step of the user and an end of the step of the user, based on the sensor data, and may estimate the step time during one step of the user, based on the detected start and end of the step. In an embodiment, the processor 710 may measure a time interval between points where the left and right hip joint angles measured through the angle sensor of the wearable device 100 intersect with each other or a time interval between points where the angular velocity of the left and right hip joints calculated from the left and right hip joint angles intersects with each other and may determine the measured time interval to be the step time of the user. In another embodiment, the processor 710 may recognize a timepoint when the user's foot touches the ground and a timepoint when the user's foot takes off from the ground, based on the sensor data measured by the IMU of the wearable device 100, and may determine a time interval between the recognized timepoints to be the step time of the user. Yet another embodiment, for the estimation of the step time, the processor 710 may use a step time estimation model trained by using, as training data, the sensor data (or the sensor data measured by the IMU) on the hip joint angles and timepoint data on the actual timepoints when the user's foot touches the ground and the user's foot takes off from the ground during walking. The step time estimation model may be a neural network model trained according to the machine learning process or linear regression algorithm described above.

In an embodiment, the processor 710 may estimate one or more other gait indices based on the walking speed and the step time. In this case, the other gait indices refer to gait indices other than the walking speed and the step time described above. The other gait indices may include, for example, at least one of a step length of one step, a stride length of two steps, a walking distance, a gait symmetry index, a gait variability index, and a walk ratio. The processor 710 may estimate the other gait indices in real time.

In an embodiment, the processor 710 may determine the step/stride length based on the sensor data. The processor 710 may estimate an average walking speed during one step of the user, based on the sensor data, and determine the step length of the step, based on the average walking speed and the step time of the step. The processor 710 may determine the step length of the step by multiplying the average walking speed by the step time during the step. In an embodiment, the processor 710 may estimate the step length of the step by using a model trained by the machine learning process or the linear regression algorithm, based on training data (e.g., the sensor data and the step length of an actual step).

In an embodiment, the processor 710 may estimate a left step length of the user and a right step length of the user, based on a first hip joint angle value when a hip joint angle of a right leg of the user is at the maximum, a second hip joint angle value when a hip joint angle of a left leg of the user is at the maximum, and the stride length of two steps of the user.

In an embodiment, the processor 710 may determine the gait symmetry index based on the sensor data. The gait symmetry index may be a value indicating how symmetrical a gait (corresponding to a right step) by the right leg of the user and a gait (corresponding to a left step) by the left leg of the user are. The processor 710 may determine an average value of the left step length of the user and an average value of the right step length of the user, based on the sensor data. The processor 710 may determine the gait symmetry index for the gait motion of the user, based on the average value of the left step length and the average value of the right step length. The processor 710 may determine the left step length and the right step length, based on the estimation of the step length described above, and may determine the gait symmetry index based on a difference between the average value of the left step length and the average value of the right step length. When the difference is large, the gait symmetry index may decrease, and, when the difference is small, the gait symmetry index may increase, but examples are not limited thereto. In another embodiment, the processor 710 may determine an average step time of the left step of the user and an average step time of the right step of the user, based on the sensor data. The processor 710 may determine the gait symmetry index for the gait motion of the user, based on the average step time of the left step and the average step time of the right step. The processor 710 may determine the average step time of the left step and the average step time of the right step, based on the estimation of the step time described above, and may determine the gait symmetry index based on a difference between the average step time of the left step and the average step time of the right step.

In an embodiment, the processor 710 may determine the gait variability index based on the sensor data. The gait variability index may be an index indicating the regularity or variability of repetitive gait motion of the user. The processor 710 may determine the gait variability index based on a variability index, such as a standard deviation of the step length or a standard deviation of a stride time of two steps. For example, the processor 710 may determine the gait variability index for the gait motion of the user, based on the standard deviation of the stride length of two steps measured during the predefined number of steps or the standard deviation of the stride time of two steps measured during the predefined number of steps during the gait motion of the user. When the standard deviation is large, the gait variability index may increase, and, when the standard deviation is small, the gait variability index may decrease, but examples are not limited thereto.

In an embodiment, the processor 710 may determine the walk ratio by dividing the average value of the step length of one step of the user by steps per minute. The steps per minute may be determined based on the average value of the step time of the step. In another embodiment, the processor 710 may determine the walk ratio by dividing an average value of the walking speed of the user by a square value of the steps per minute. The walk ratio may be used as an indicator to estimate the likelihood of the user's fall. Yet another embodiment, for the estimation of the walk ratio, the processor 710 may use a walk ratio estimation model trained by using training data including the sensor data (or the sensor data measured by the IMU and/or the angle sensor) and information on an actual walk ratio. The walk ratio estimation model may be a neural network model trained according to the machine learning process or linear regression algorithm described above.

The processor 710 may determine a gait score corresponding to a gait evaluation result of the gait motion of the user, based on the gait indices estimated as described above. In an embodiment, the processor 710 may determine the gait score based on the walking speed, the walk ratio, the gait symmetry index, and the gait variability index. For example, the processor 710 may determine an average, sum, or weighted sum of the walking speed, the walk ratio, the gait symmetry index, and the gait variability index to be the gait score, but examples are not limited thereto. The processor 710 may generate gait evaluation information including the gait score and one or more gait indices. The generated gait evaluation information may be provided to the user through the display module 740 and/or the sound output module 750. According to embodiments, the gait evaluation information may be provided to the user through the wearable device 100.

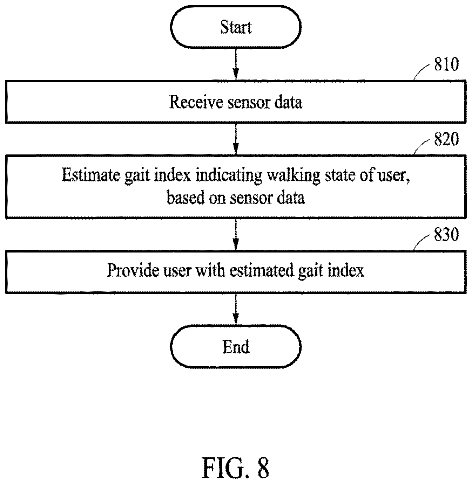

FIG. 8 is a flowchart illustrating a method of providing a gait index of a user wearing a wearable device, according to an embodiment. According to an embodiment, the method of providing the gait index may be performed by the electronic device 210.

Referring to FIG. 8, in operation 810, the electronic device 210 may receive sensor data including the motion information of a user wearing a wearable device 100 from the wearable device 100. The sensor data may include sensor data on acceleration and rotation velocity according to the body motion of the user and sensor data on a joint angle (e.g., a hip joint angle) of the user.

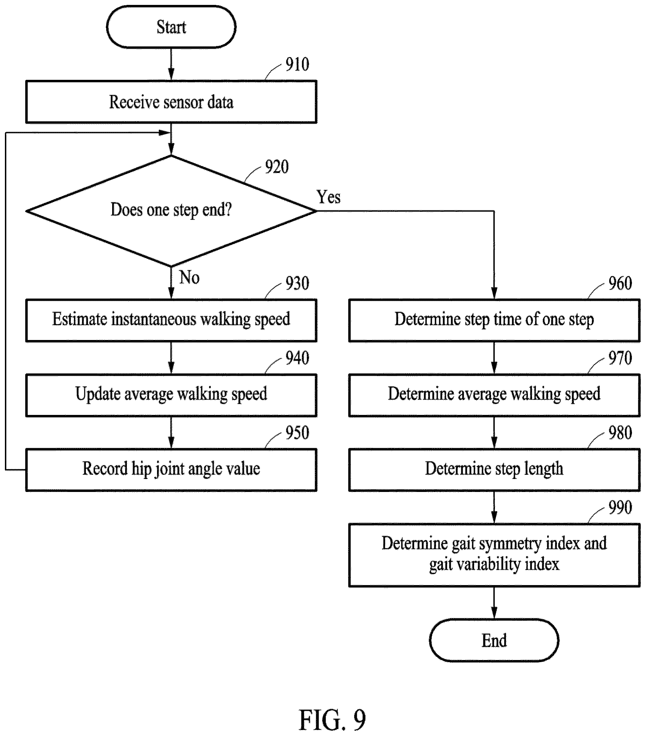

In operation 820, the electronic device 210 may estimate the gait index indicating a walking state of the user, based on the sensor data. In an embodiment, the electronic device 210 may estimate the walking speed of the user by using a walking speed estimation model with the sensor data as an input. The electronic device 210 may estimate a step time of the user, based on the sensor data, and may estimate other gait indices based on the walking speed and the step time. The electronic device 210 may estimate the other gait indices including, for example, at least one of a step length of one step, a stride length of two steps, a walking distance, a gait symmetry index, a gait variability index, and a walk ratio. For example, the electronic device 210 may determine the walk ratio by dividing an average value of the step length of one step of the user by the number of steps per minute or by dividing an average value of the walking speed of the user by a square value of the number of steps per minute. The process of estimating each of the gait indices is described in detail with reference to FIG. 9.

In operation 830, the electronic device 210 may provide the user with the estimated gait index. The providing of the gait index to the user may include an operation of determining a gait score corresponding to a gait evaluation result of the gait motion of the user, based on the estimated gait index, and an operation of providing the user with gait evaluation information including the determined gait score and the estimated gait index. The electronic device 210 may provide the user with the estimated gait index through a display module and/or a sound output module. In an embodiment, the user may view gait indices through a program (e.g., an application) installed in the electronic device 210.

The electronic device 210 may determine the gait score corresponding to the gait evaluation result of the gait motion of the user, based on the estimated gait index, and may provide the user with the gait evaluation information including the determined gait score and the estimated one or more gait indices. The electronic device 210 may determine the gait score based on the walking speed, the walk ratio, the gait symmetry index, and the gait variability index. The walking speed is a representative gait index generally decreasing with aging. The gait symmetry index and the gait variability index may not be good when there is a physical abnormality in a musculoskeletal or nervous system. The gait variability index tends to decrease with aging and is related to the likelihood of falls. In an embodiment, the gait score may be determined based on an average or a weighted average of such gait indices (e.g., the walking speed, the gait symmetry index, the gait variability index, and the walk ratio), and the determined gait score may be provided to the user. In an embodiment, there may be a correlation that the gait score increases while the walk ratio increases and the gait symmetry index and the gait variability index decrease.

FIG. 9 is a flowchart illustrating a method of estimating a gait index, according to an embodiment. According to an embodiment, the method of estimating the gait index may be performed by the electronic device 210. In an embodiment, at least one of the operations of FIG. 9 may be simultaneously or parallelly performed with one another, and the order of the operations may be changed. In addition, at least one of the operations may be omitted or another operation may be additionally performed.

Referring to FIG. 9, in operation 910, the electronic device 210 may receive sensor data including the motion information of a user wearing a wearable device 100 from the wearable device 100. In the process of the user walking while wearing the wearable device 100, an IMU embedded in the wearable device 100 may measure acceleration and rotation velocity around the user's pelvis in each of up and down, forward and backward, and left and right directions and may output measured sensor data. The sensor data output from the IMU may include values of the acceleration in each of three axes and values of the rotation velocity in each of the three axes.

In operation 920, the electronic device 210 may determine whether one step of the user ends, based on the sensor data received from the wearable device 100. In an embodiment, the electronic device 210 may determine whether one step ends by recognizing the start and end of the step based on the sensor data. The criteria for determining the start and end of the step may be determined arbitrarily. For example, the moment when the user's foot touches the ground or the moment when the user's foot takes off from the ground may both be the determination criteria. For another example, the moment when left and right hip joint angle values intersect with each other or the moment when left and right hip joint angular velocity values intersect with each other may be the determination criteria. In an embodiment, the electronic device 210 may estimate the start and end of one step by using a model trained to distinguish the start and end of the step when the sensor data is input.

When the step of the user does not end ('no' in operation 920), the electronic device 210, in operation 930, may estimate instantaneous walking speed of the user, based on the sensor data. In an embodiment, the sensor data obtained by the IMU and/or an angle sensor of the wearable device 100 may be input to the walking speed estimation model described above, and the electronic device 210 may obtain a walking speed estimation value corresponding to the instantaneous walking speed of the user through the walking speed estimation model. The walking speed estimation model may output the walking speed estimation value continuously within a time interval of one step.

In operation 940, the electronic device 210 may update an average walking speed for the step of the user, based on the instantaneous walking speed. The electronic device 210 may update the average walking speed by averaging cumulative instantaneous walking speed from the start of the step to a current timepoint, for example.

In operation 950, the electronic device 210 may record a hip joint angle value based on the angle sensor. In an embodiment, operation 950 may be performed selectively. The electronic device 210 may record a hip joint angle value of a front leg and a hip joint angle value of a rear leg when the hip joint angle value of the rear leg is at the maximum during one step. The hip joint angle values recorded as such may be used to determine a step length of the user.

When the step of the user ends ('yes' in operation 920), the electronic device 210, in operation 960, may determine a step time of the step of the user, based on the sensor data. For example, as described in operation 920, the electronic device 210 may detect the start of the step of the user and the end of the step of the user, based on the sensor data, and may estimate the step time during the step of the user, based on the detected start and end of the step.

In operation 970, the electronic device 210 may determine the average walking speed of the user. The electronic device 210 may perform the update process of the average walking speed in operation 940 on a time interval of one step and may determine the average walking speed for the step as a performing result.

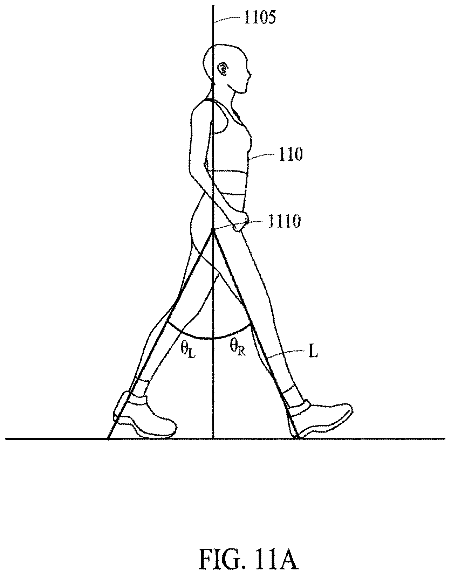

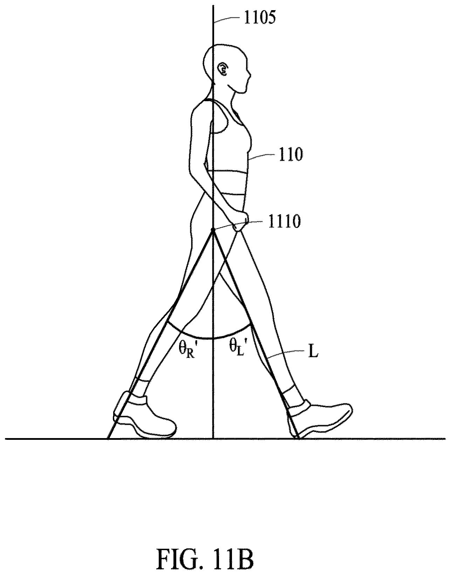

In operation 980, the electronic device 210 may determine the step length of the user. The electronic device 210 may determine the step length of the step by multiplying the average walking speed during the step by the step time of the step, for example. For another example, the electronic device 210 may determine the step length by using a model trained to estimate the step length based on the sensor data. Yet another example, the electronic device 210 may estimate a left step length of the user and a right step length of the user, based on a first hip joint angle value when a hip joint angle of a right leg of the user is at the maximum, a second hip joint angle value when a hip joint angle of a left leg of the user is at the maximum, and a stride length of two steps of the user. The method of estimating the left step length and the right step length based on the hip joint angle values is described in detail below with reference to FIGS. 11A and 11B.

In operation 990, the electronic device 210 may determine a gait symmetry index and a gait variability index. The gait symmetry index may correspond to an index indicating a difference between the left step length and the right step length or a difference between a step time of a left step and a step time of a right step. The electronic device 210 may calculate an average value of the left step length and an average value of the right step length for steps of the user, for example, and may determine the gait symmetry index based on a difference between the average value of the left step length and the average value of the right step length. For another example, the electronic device 210 may determine the gait symmetry index based on a difference between the step time of the left step and the step time of the right step for the steps of the user.

The electronic device 210 may determine the gait variability index for the gait motion of the user, based on a standard deviation of a stride length of two steps measured during the predefined number (e.g., 10 or 20 steps) of steps or a standard deviation of a stride time of two steps measured during the predefined number of steps during the gait motion of the user. For example, the electronic device 210 may determine a result value of dividing the standard deviation for the stride length of two steps by an average of the stride length of two steps or a result value of dividing the standard deviation for the stride time of two steps by an average of the stride time of two steps to be the gait variability index. For another example, the electronic device 210 may determine the standard deviation for the stride length of two steps or the standard deviation for the stride time of two steps to be the gait variability index. Yet another example, the electronic device 210 may determine a difference between a maximum value and a minimum value for stride lengths of two steps or stride times of two steps measured during the predefined number of steps or a difference between an upper value and a lower value of a certain percentage to be the gait variability index.

In an embodiment, the electronic device 210 may determine a walk ratio by dividing an average value of the step length of one step of the user by the number of steps per minute or by dividing an average value of the walking speed of the user by a square value of the number of steps per minute. In this case, the steps per minute may correspond to (60 / an average value of the step time of the step).

FIG. 10 is a diagram illustrating a stride length according to an embodiment.

FIG. 10 illustrates a first position 1010 at a first timepoint when a user's right foot touches the ground, a second position 1020 at a second timepoint when the user's left foot touches the ground after the first timepoint, a third position 1012 at a third timepoint when the right foot touches the ground again after the second timepoint, and a fourth position 1022 at a fourth timepoint when the left foot touches the ground again after the third timepoint. A length from the first position 1010 where the user's right foot touches the ground to the second position 1020 where the user's left foot touches the ground may correspond to a left step length of the user, and a length from the second position 1020 where the user's left foot touches the ground to the third position 1012 where the user's right foot touches the ground may correspond to a right step length of the user. The left step length and the right step length may both correspond to a step length of one step and a stride length from when the right foot (or the left foot) touches the ground to when the right foot (or the left foot) touches the ground again may correspond to the stride length of two steps. The stride length of two steps may correspond to a sum of the left step length and the right step length.

FIGS. 11A and 11B are diagrams each illustrating an operation of estimating a stride length based on a hip joint angle value, according to an embodiment.

Referring to FIGS. 11A and 11B, when a ratio that each of a right step length and a left step length occupies of a stride length of two steps is calculated, the right step length and the left step length may be estimated.

The right step length may be determined based on FIG. 11A and Equation 1 below.

Equation 1

$$Right\ step\ length = L \times (sin\theta_L + sin\theta_R)$$

In this case, L may correspond to a right leg length and a left leg length from a position 1110 of a hip joint of a user. $\theta_L$ may be a hip joint angle when a hip joint angle of the left leg of the user is at the maximum backward among hip joint values formed by the left leg of the user and a line 1105 that is perpendicular to the ground and passes the position 1110 of the hip joint in a walking process of the user. $\theta_R$ may be a hip joint angle when a hip joint angle of the right leg of the user is at the maximum forward among hip joint values formed by the right leg of the user and the line 1105 in the walking process of the user. $\theta_L$ and $\theta_R$ are both assumed to have positive values.

The left step length may be determined based on FIG. 11B and Equation 2 below.

Equation 2

$$Left\ step\ length = L \times (sin\theta_L{}' + sin\theta_R{}')$$

In this case, L may correspond to a right leg length and a left leg length from a position 1110 of a hip joint of a user. $\theta_L{}'$ may be a hip joint angle when the hip joint angle of the left leg of the user is at the maximum forward among hip joint values formed by the left leg of the user and the line 1105 that is perpendicular to the ground and passes the position 1110 of the hip joint in the walking process of the user. $\theta_R{}'$ may be a hip joint angle when the hip joint angle of the right leg of the user is at the maximum backward among hip joint values formed by the right leg of the user and the line 1105 in the walking process of the user. $\theta_L{}'$ and $\theta_R{}'$ are both assumed to have positive values.

The right step length may be determined based on a stride length of two steps and a hip joint angle value as shown in Equation 3 below.

Equation 3

$$Right\ step\ length$$
$$= Stride\ length\ of\ two\ steps \times ((sin\theta\_L + sin\theta\_R))/((sin\theta\_L + sin\theta\_R\ )$$
$$+ (sin\theta\_L' + sin\theta\_R'))$$

The left step length may be determined based on the stride length of two steps and the hip joint angle value as shown in Equation 4 below.

Equation 4

$$Left\ step\ length$$
$$= Stride\ length\ of\ two\ steps \times ((sin\theta\_L' + sin\theta\_R'))/((sin\theta\_L + sin\theta\_R\ )$$
$$+ (sin\theta\_L' + sin\theta\_R'))$$

A wearable device (e.g., the wearable device 100 of FIGS. 1 and 3, or the wearable device 100 of FIG. 2) may estimate the right step length and the left step length from the stride length of two steps and the hip joint angle value by using Equations 3 and 4.

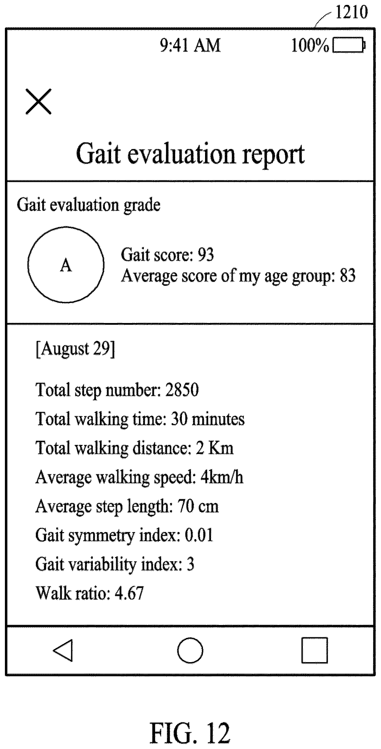

FIG. 12 is a diagram illustrating a screen providing gait evaluation information, according to an embodiment.

Referring to FIG. 12, the electronic device 210 may provide a UI screen 1210 for providing the gait evaluation information of a user. The gait evaluation information may include information on one or more estimated gait indices and a gait score determined based on the gait indices. In an embodiment, the electronic device 210 may provide the gait indices including at least one of a total number of steps, a spent walking time, an (average) walking speed, a total walking distance, an (average) step/stride length, a gait symmetry index, a gait variability index, and a walk ratio through the UI screen 1210.

In an embodiment, the electronic device 210 may update the average step/stride length of one or more steps in real time and provide the user with the updated average step/stride length through the UI screen 1210 or may provide the user with voice data for informing the average step/stride length through an audio device. In an embodiment, the electronic device 210 may calculate a relative percentile score in a user group to which the user's age or gender belongs for each of the gait indices including the walking speed, the walk ratio, the gait symmetry index, and the gait variability index, and may provide the user with the calculated percentile score of a weighted average or an average of each of the gait indices. The electronic device 210 may collect gait indices of a user group of an age group similar to the user's age and may calculate a percentile position in the user group for a gait index of the user. For example, under the assumption that, when the walking speed of a 65-year-old user is the fastest compared to the walking speed of a user group of a 60 to 70 age group to which the user's age belongs, the percentile score of the user is 100, and, when the walking speed of the user is the slowest, the percentile score is 0, the percentile score corresponding to the user may be determined based on which percentile position the walking speed of the user corresponds to.

FIG. 13 is a diagram illustrating a configuration and operation of a training device for training a walking speed estimation model, according to an embodiment.

Referring to FIG. 13, a training device 1300 may be a device for training the walking speed estimation model through a linear regression method or a machine learning method. The training device 1300 may train the walking speed estimation model based on the sensor data of the wearable device 100 obtained during the walking of a user and actual walking speed data.

The training device 1300 may include a processor 1310 and a memory 1320. The processor 1310 may control at least one other component (e.g., a hardware or software component) of the training device 1300 and may perform various types of data processing or operations. The processor 1310 may include a main processor (e.g., a CPU or an AP) or an auxiliary processor (e.g., a GPU, an NPU, an ISP, a sensor hub processor, or a CP) that is operable independently of or in conjunction with the main processor. The memory 1320 may store various pieces of data used by at least one component (e.g., the processor 1310) of the training device 1300. The memory 1320 may include at least one instruction executable by the processor 1310. The memory 1320 may include a volatile memory or a non-volatile memory.

The processor 1310 may process (e.g., preprocess) training data collected to train the walking speed estimation model. The training data may include, for example, velocity data of a treadmill and sensor data obtained by a sensor (e.g., an angle sensor or an IMU) of the wearable device 100 when the user walks at a certain walking speed while letting the user walk at various walking speeds (e.g., 0 km/h, 0.5 km/h, 1.0 km/h, etc.) on the treadmill with wearing the wearable device 100. The processor 1310 may remove noise, which removes sensor data obtained during a time other than the time when the user walks at a certain walking speed and sensor data obtained while the user stands still, from the collected training data. The processor 1310 may perform data normalization for each type of sensor data on the training data from which the noise is removed.

The processor 1310 may select a type of the walking speed estimation model on which training is performed. For example, a CNN model, a linear regression model, or a model in which a fully connected layer is connected to an output unit of an LSTM model may be used as the walking speed estimation model, but examples are not limited to the foregoing models.

The processor 1310 may select an input feature to be used for the training of the walking speed estimation model. The processor 1310 may specify input data of the walking speed estimation model. For example, the processor 1310 may select, as the input feature, angular velocity data (e.g., angular velocity data of each of an x-axis, a y-axis, and a z-axis) and acceleration data (e.g., acceleration data of each of the x-axis, the y-axis, and the z-axis) obtained from the IMU of the wearable device 100 or may select, as the input feature, angular velocity data (e.g., angular velocity data corresponding to a right hip joint or angular velocity data corresponding to a left hip joint) of a hip joint obtained from the angle sensor of the wearable device 100, the angular velocity data, and the acceleration data. Alternatively, the processor 1310 may select, as the input feature, the acceleration data, the angular velocity data obtained from the IMU, rotational angle data (e.g., a joint angle of the right hip joint or a joint angle of the left hip joint), and the angular velocity data of the hip joint obtained from the angle sensor.

The processor 1310 may input the selected input feature to the walking speed estimation model, may calculate a difference between the actual walking speed value (e.g., the speed of the treadmill) and a walking speed estimation value output from the walking speed estimation model, and may update parameters of the walking speed estimation model such that the difference may decrease. For example, the processor 1310 may perform the update process through an error backpropagation algorithm. The processor 1310 may perform the training process of the walking speed estimation model repeatedly as many as the number of epochs determined for training. The parameters of the walking speed estimation model may be updated for each epoch.

When the training process is completed, the walking speed estimation model of which the parameters are updated may be embedded in the electronic device 210 and/or the wearable device 100. The walking speed estimation model may estimate the walking speed of the user in real time, based on sensor data corresponding to the type of the input feature selected in the training process of the walking speed estimation model among various types of sensor data measured from the IMU and the angle sensor of the wearable device 100.

FIG. 14 is a flowchart illustrating operations of a training method of a walking speed estimation model, according to an embodiment. According to an embodiment, the training method may be performed by a training device (e.g., the training device 1300 of FIG. 13).

Referring to FIG. 14, in operation 1410, training data for the training of the walking speed estimation model may be collected. In an embodiment, when a user wearing the wearable device 100 walks at various walking speeds on a treadmill, sensor data obtained from an angle sensor and IMU of the wearable device 100 may be collected. For the collection of the training data, the user may walk 10 steps on the treadmill at each walking speed: 0 km/h, 0.5 km/h, 1.0 km/h, ..., 6.5 km/h, 7.0 km/h. The training data may be collected in each operation state (e.g., a state in which a walking assistance mode is active, a state in which an exercise assistance mode is active, or a state in which the walking assistance mode or the exercise assistance mode is inactive) of the wearable device 100.

The sensor data may be collected at every sampling time (e.g., 10 ms). The collected sensor data may include, for example, velocity data of the treadmill corresponding to actual walking speed of the user and torque data on a torque value generated by a driving module of the wearable device 100, sensor data (e.g., angular velocity data corresponding to a right hip joint, angular velocity data corresponding to a left hip joint, a joint angle of the right hip joint, or a joint angle of the left hip joint) of the angle sensor of the wearable device 100, or sensor data (e.g., acceleration data of each of an x-axis, a y-axis, and a z-axis, gyro sensor data, or Euler angle data) of the IMU of the wearable device 100.

In operation 1420, the training device may perform preprocessing on the collected training data. The training device may perform noise removal (or outlier elimination), which removes sensor data obtained during a time other than a time when the user walks at a certain walking speed and sensor data obtained while the user stands still, from the collected training data. The training device may perform data normalization on the training data from which the noise is removed. The training device, for example, may calculate an average and a standard deviation for each type of each piece of data included in the training data and may normalize the data for each data type by using the calculated average and standard deviation.

In operation 1430, the training device may train the walking speed estimation model based on the training data on which the preprocessing is performed. For example, a CNN model, a linear regression model, or a model in which a fully connected layer is connected to an output unit of an LSTM model may be used as the walking speed estimation model, but examples are not limited to the foregoing models. The model in which a fully connected layer is connected to an output unit of an LSTM model may be a model implemented such that a walking speed estimation value in a hidden state form inside the LSTM model may be output to the outside through the fully connected layer connected to the LSTM model.

The training device may select, as an input feature, sensor data to be used for the training of the walking speed estimation model among various types of sensor data obtained from the wearable device 100. For example, (1) the sensor data of the IMU, (2) the sensor data of the angle sensor, or (3) any one of the sensor data of the IMU and the sensor data of the angle sensor may be selected as an input feature.

The training device may initially determine a hyperparameter for the training of the walking speed estimation model. For example, the determined hyperparameter may include a loss type (e.g., a mean square error), a learning rate, the number of epochs, the number of hidden states of the walking speed estimation model, a batch size, or the like.

The training device may repeatedly perform training as many as the number of epochs determined based on the training data. During the training, the training device may update parameters (e.g., a weight) of the walking speed estimation model at every epoch. For example, the training device may calculate a difference by comparing an actual walking speed value with a walking speed estimation value output by the walking speed estimation model according to an error backpropagation algorithm and may update parameters of the walking speed estimation model such that the difference may decrease.

In an embodiment, the training device may perform a verification process of a training result when the training of the walking speed estimation model is completed. For example, the training device may divide the entire data into 10 groups, may use data of one group thereof as verification data, and may use data of the remaining 9 groups as training data. The training device may perform training based on the training data, and then, may obtain the walking speed estimation value by inputting the verification data to the walking speed estimation model and may compare the obtained walking speed estimation value with the actual walking speed value. The training device may determine the accuracy of the walking speed estimation model based on a comparison result. As the walking speed estimation value is more similar to the actual walking speed value, the accuracy may be determined to be higher. When the accuracy of the walking speed estimation model is lower than a required level, the training device may perform the training and verification processes again by changing the selection of the input feature input to the walking speed estimation model or changing the hyperparameter. As a result of verification, when the accuracy of the walking speed estimation model meets the required level, the training device may perform training by using the entire data (e.g., the data of 10 groups) as the training data by using the hyperparameter used for the training.

In operation 1440, the walking speed of the user may be estimated by using the trained walking speed estimation model. When the training process is completed, the walking speed estimation model may estimate the walking speed of the user, based on sensor data corresponding to the type of the input feature selected in the training process of the walking speed estimation model among various types of sensor data measured from the IMU and angle sensor of the wearable device 100. When the walking speed estimation model is the model in which a fully connected layer is connected to an output unit of an LSTM model, a vector, obtained at a time t, of the selected input feature of the wearable device 100 may be input to the walking speed estimation model, and, as the vector of the input feature is applied to a weight matrix, determined through the training process, of the LSTM model, a walking speed estimation value may be finally output at the time t from the walking speed estimation model. The estimation of the walking speed may be performed in the electronic device 210 and/or the wearable device 100.

As described above, the process of estimating a gait index described herein may also be performed in the wearable device 100 as well as the electronic device 210. For example, the processor 512 of the wearable device 100 may estimate the walking speed of the user in real time by inputting sensor data obtained by the sensor modules 520 and 520-1 to the walking speed estimation model. In this case, the sensor data may include at least one of a hip joint angle value (e.g., angular velocity or rotation velocity), measured by the angle sensor 125, of the user and a motion value (e.g., acceleration or rotation velocity) measured by the IMU 135. The wearable device 100 may transmit the estimated walking speed to the electronic device 210 through the communication module 516.

It should be appreciated that various embodiments of the present disclosure and the terms used therein are not intended to limit the technological features set forth herein to particular embodiments and include various changes, equivalents, or replacements for a corresponding embodiment. In connection with the description of the drawings, like reference numerals may be used for similar or related components. It is to be understood that a singular form of a noun corresponding to an item may include one or more of the things unless the relevant context clearly indicates otherwise. As used herein, "A or B", "at least one of A and B", "at least one of A or B", "A, B or C", "at least one of A, B and C", and "A, B, or C," each of which may include any one of the items listed together in the corresponding one of the phrases, or all possible combinations thereof. Terms such as "first", "second", or "first" or "second" may simply be used to distinguish the component from other components in question, and do not limit the components in other aspects (e.g., importance or order). It is to be understood that if an element (e.g., a first element) is referred to, with or without the term "operatively" or "communicatively," as "coupled with," "coupled to," "connected with," or "connected to" another element (e.g., a second element), the element may be coupled with the other element directly (e.g., by wire), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

The software may include a computer program, a piece of code, an instruction, or some combination thereof, to independently or collectively instruct or configure the processing device to operate as desired. Software and data may be embodied permanently or temporarily in any type of machine, component, physical or virtual equipment, or computer storage medium or device capable of providing instructions or data to or being interpreted by the processing device. The software also may be distributed over network-coupled computer systems so that the software is stored and executed in a distributed fashion. The software and data may be stored by one or more non-transitory computer-readable recording mediums. Embodiments as set forth herein may be implemented as software including one or more instructions that are stored in a storage medium (e.g., the memory 514) that is readable by a machine. For example, a processor of the machine may invoke at least one of the one or more instructions stored in the storage medium and execute it. This allows the machine to be operated to perform at least one function according to the at least one instruction invoked. The one or more instructions may include code generated by a compiler or code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to one embodiment, a method according to embodiments of the disclosure may be included and provided in a computer program product. The computer program product may be traded as a product between a seller and a buyer. The computer program product may be distributed in the form of a machine-readable storage medium (e.g., a compact disc read-only memory (CD-ROM)), or be distributed (e.g., downloaded or uploaded) online via an application store (e.g., PlayStore™), or between two user devices (e.g., smartphones) directly. If distributed online, at least part of the computer program product may be temporarily generated or at least temporarily stored in the machine-readable storage medium, such as a memory of the manufacturer's server, a server of the application store, or a relay server.

According to an embodiment, each component (e.g., a module or a program) of the above-described components may include a single entity or multiple entities, and some of the multiple entities may be separately disposed in different components. According to an embodiment, one or more of the above-described components may be omitted, or one or more other components may be added. Alternatively or additionally, a plurality of components (e.g., modules or programs) may be integrated into a single component. In such a case, according to an embodiment, the integrated component may still perform one or more functions of each of the components in the same or similar manner as they are performed by a corresponding one among the components before the integration. According to embodiments, operations performed by the module, the program, or another component may be carried out sequentially, in parallel, repeatedly, or heuristically, or one or more of the operations may be executed in a different order or omitted, or one or more other operations may be added.

**Claims**

1. An electronic device 210 comprising:

   a communication module 730 configured to receive sensor data comprising motion information of a user wearing a wearable device 100 from the wearable device 100;
   a processor 710 configured to estimate a gait index indicating a walking state of the user, based on the sensor data; and
   a display module 740 configured to output physical ability information comprising the gait index, wherein
   the processor 710 is further configured to
   estimate a walking speed of the user by using a walking speed estimation model with the sensor data as an input,
   estimate a step time of the user, based on the sensor data, and
   estimate another gait index based on the walking speed and the step time.

2. The electronic device of claim 1, wherein

   the processor 710 is further configured to,
   based on the walking speed and the step time, estimate the other gait index comprising at least one of a step length of one step, a stride length of two steps, a walking distance, a gait symmetry index, a gait variability index, and a walk ratio.

3. The electronic device of claim 1 or 2, wherein

   the processor 710 is further configured to
   detect a start of a step and an end of the step, based on the sensor data, and estimate a step time during one step of the user, based on the start of the step and the end of the step.

4. The electronic device of claim 3, wherein

   the processor 710 is further configured to
   estimate an average walking speed during one step of the user, based on the sensor data, and determine a step length of the step, based on the average walking speed and the step time of the step.

5. The electronic device of any one of claims 2 to 4, wherein

   the processor 710 is further configured to
   determine an average value of a left step length of the user and an average value of a right step length of the user, based on the sensor data, and determine the gait symmetry index for gait motion of the user, based on the average value of the left step length and the average value of the right step length.

6. The electronic device of any one of claims 2 to 5, wherein

   the processor 710 is further configured to
   determine an average step time of a left stride of the user and an average step time of a right stride of the user, based on the sensor data, and determine the gait symmetry index for gait motion of the user, based on the average step time of the left stride and the average step time of the right stride.

7. The electronic device of any one of claims 2 to 6, wherein

   the processor 710 is further configured to

determine the gait variability index for the gait motion of the user, based on a standard deviation of a stride length of two steps measured during the predefined number of steps or a standard deviation of a stride time of two steps measured during the predefined number of steps during the gait motion of the user.

8. The electronic device of any one of claims 2 to 7, wherein

the processor 710 is further configured to
determine the walk ratio by dividing an average value of a step length of one step of the user by steps per minute.

9. The electronic device of claim 8, wherein

the processor 710 is further configured to
determine the steps per minute based on an average value of a step time of the step.

10. The electronic device of any one of claims 2 to 9, wherein

the processor 710 is further configured to
determine the walk ratio by dividing an average value of the walking speed of the user by a square value of the steps per minute.

11. The electronic devices of any one of claims 1 to 10, wherein

the processor 710 is further configured to
estimate a left step length of the user and a right step length of the user, based on a first hip joint angle value when a hip joint angle of a right leg of the user is at a maximum, a second hip joint angle value when a hip joint angle of a left leg of the user is at a maximum, and a stride length of two steps of the user.

12. The electronic device of any one of claims 2 to 11, wherein

the processor 710 is further configured to
determine a gait score corresponding to a gait evaluation result of the gait motion of the user, based on the estimated gait index, and control to provide the user with the gait evaluation result comprising the determined gait score and the estimated one or more gait indices.

13. The electronic device of any one of claims 1 to 12, wherein
rotation velocity information and acceleration information obtained from an inertial measurement unit of the wearable device 100 is input to the walking speed estimation model, and the walking speed estimation model is configured to output an estimated walking speed value of the user, based on the rotation velocity information and the acceleration information.

14. A method of estimating a gait index of a user wearing a wearable device 100,
the method comprising:

receiving sensor data comprising motion information of the user wearing the wearable device 100 from the wearable device 100;
estimating the gait index indicating a walking state of the user, based on the sensor data; and
providing the estimated gait index to the user, wherein
the estimating the gait index comprises:

estimating a walking speed of the user by using a walking speed estimation model with the sensor data as an input;
estimating a step time of the user, based on the sensor data; and
estimating another gait index based on the walking speed and the step time.

15. A non-transitory computer-readable storage medium storing instructions that, when executed by a processor, cause the processor to perform the method of claim 14.

FIG. 1

FIG. 2

FIG. 3

FIG. 4

500

Sensor module
520

510

Memory
514

Processor
512

Battery
540

Communication
module 516

530

Motor
534

Motor driver circuit
532

FIG. 5A

FIG. 5B

210

Electronic device

Control command

Wearable device
100

212

Control result

Sensor data

210

Electronic device

Wearable device
100

212

## FIG. 6

210

```
┌─────────────────────────────────────────────────┐
│   ┌──────────────┐          ┌──────────────┐     │
│   │              │   ◄────►  │   Memory     │     │
│   │              │          │   720        │     │
│   │              │          └──────────────┘     │
│   │              │                               │
│   │  Processor   │   ◄────►  ┌──────────────┐     │
│   │  710         │          │Communication │     │
│   │              │          │module 730    │     │
│   │              │          └──────────────┘     │
│   │              │   ◄────►  ┌──────────────┐     │
│   │              │          │Display module│     │
│   │              │          │   740        │     │
│   └──────────────┘          └──────────────┘     │
│         ▲                                         │
│         │                                         │
│         ▼                         ◄──────┐        │
│   ┌──────────────┐          ┌──────────────┐     │
│   │ Sound output │          │ Input module │     │
│   │ module 750   │          │   760        │     │
│   └──────────────┘          └──────────────┘     │
└─────────────────────────────────────────────────┘
```

FIG. 7

```
              ┌──────────────┐
              │    Start     │
              └──────┬───────┘
                     │                          ╱ 810
     ┌───────────────▼────────────────────────┐
     │          Receive sensor data            │
     └───────────────┬────────────────────────┘
                     │                          ╱ 820
     ┌───────────────▼────────────────────────┐
     │ Estimate gait index indicating walking  │
     │    state of user, based on sensor data  │
     └───────────────┬────────────────────────┘
                     │                          ╱ 830
     ┌───────────────▼────────────────────────┐
     │    Provide user with estimated gait     │
     │                index                    │
     └───────────────┬────────────────────────┘
                     │
              ┌──────▼───────┐
              │     End      │
              └──────────────┘
```

# FIG. 8

```
                    ┌─────────────┐
                    │    Start    │
                    └─────────────┘
                           │
                           ▼                    ⌐910
              ┌────────────────────────────┐
              │     Receive sensor data    │
              └────────────────────────────┘
                           │
                           ▼                    ⌐920
                      ◇ Does one        Yes ──────────────────┐
                        step end? ◇                           │
                           │                                  │
                          No          ⌐930                    │         ⌐960
                           ▼                                  ▼
              ┌────────────────────────────┐    ┌────────────────────────────┐
              │ Estimate instantaneous     │    │ Determine step time of      │
              │ walking speed              │    │ one step                    │
              └────────────────────────────┘    └────────────────────────────┘
                           │          ⌐940                    │         ⌐970
                           ▼                                  ▼
              ┌────────────────────────────┐    ┌────────────────────────────┐
              │ Update average walking     │    │ Determine average walking   │
              │ speed                      │    │ speed                       │
              └────────────────────────────┘    └────────────────────────────┘
                           │          ⌐950                    │         ⌐980
                           ▼                                  ▼
              ┌────────────────────────────┐    ┌────────────────────────────┐
              │ Record hip joint angle     │    │ Determine step length       │
              │ value                      │    └────────────────────────────┘
              └────────────────────────────┘                 │         ⌐990
                                                             ▼
                                             ┌────────────────────────────┐
                                             │ Determine gait symmetry     │
                                             │ index and gait variability  │
                                             │ index                       │
                                             └────────────────────────────┘
                                                             │
                                                             ▼
                                                    ┌─────────────┐
                                                    │     End     │
                                                    └─────────────┘
```

FIG. 9

FIG. 10

FIG. 11A

FIG. 11B

1210

9:41 AM    100% ▭

✕

# Gait evaluation report

Gait evaluation grade

( A )    Gait score: 93
Average score of my age group: 83

[August 29]

Total step number: 2850

Total walking time: 30 minutes

Total walking distance: 2 Km

Average walking speed: 4km/h

Average step length: 70 cm

Gait symmetry index: 0.01

Gait variability index: 3

Walk ratio: 4.67

◁    ◯    ▢

# FIG. 12

210

Training device 1300

Processor
1310

Memory
1320

100

FIG. 13

Start

Collect training data /1410

Preprocess training data /1420

Train walking speed estimation model /1430

Estimate walking speed of user by using trained walking
speed estimation model /1440

End

FIG. 14

**EP 4 438 239 A1**

<table>
<tr><td colspan="2" align="center">INTERNATIONAL SEARCH REPORT</td><td colspan="2">International application No.<br>**PCT/KR2022/021526**</td></tr>
</table>

**A. CLASSIFICATION OF SUBJECT MATTER**

**B25J 9/00**(2006.01)i; **A61B 5/11**(2006.01)i; **A61B 5/00**(2006.01)i; **B25J 9/16**(2006.01)i; **B25J 13/08**(2006.01)i; **B25J 19/02**(2006.01)i; **A61H 1/02**(2006.01)i

According to International Patent Classification (IPC) or to both national classification and IPC

**B. FIELDS SEARCHED**

Minimum documentation searched (classification system followed by classification symbols)

B25J 9/00(2006.01); A61B 5/11(2006.01); A61H 3/00(2006.01); G01P 21/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 보행(walk), 속도(speed), 시간(time), 지수(index), 모델(model) 및 평가(evaluate)

**C. DOCUMENTS CONSIDERED TO BE RELEVANT**

| Category* | Citation of document, with indication, where appropriate, of the relevant passages | Relevant to claim No. |
|---|---|---|
| X | JP 2015-217250 A (FUJITSU LTD.) 07 December 2015 (2015-12-07)<br>See paragraphs [0012]-[0051] and [0078] and figures 1-7. | 1-4,14,15 |
| Y | | 5-13 |
| Y | KR 10-2016-0079627 A (SAMSUNG ELECTRONICS CO., LTD.) 06 July 2016 (2016-07-06)<br>See paragraphs [0013]-[0014], [0046], [0056]-[0063], [0168] and [0199]-[0204] and figures 1, 4 and 7. | 5,6,8-13 |
| Y | JP 2017-504440 A (KONINKLIJKE PHILIPS N.V.) 09 February 2017 (2017-02-09)<br>See paragraphs [0017]-[0019]. | 7 |
| A | JP 2021-003605 A (SHIINA, Kazuhiro) 14 January 2021 (2021-01-14)<br>See paragraphs [0015]-[0072] and figures 1-6. | 1-15 |
| A | KR 10-2019-0113235 A (AGENCY FOR DEFENSE DEVELOPMENT) 08 October 2019 (2019-10-08)<br>See paragraphs [0025]-[0082] and figures 1-6. | 1-15 |

☐ Further documents are listed in the continuation of Box C.   ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **22 March 2023** | **22 March 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2022/021526**

| Patent document cited in search report | | | Publication date (day/month/year) | Patent family member(s) | | | Publication date (day/month/year) |
|---|---|---|---|---|---|---|---|
| JP | 2015-217250 | A | 07 December 2015 | None | | | |
| KR | 10-2016-0079627 | A | 06 July 2016 | CN | 105726267 | A | 06 July 2016 |
| | | | | CN | 105726267 | B | 18 October 2019 |
| | | | | EP | 3037081 | A1 | 29 June 2016 |
| | | | | EP | 3037081 | B1 | 11 March 2020 |
| | | | | JP | 2016-123855 | A | 11 July 2016 |
| | | | | JP | 2020-110611 | A | 27 July 2020 |
| | | | | JP | 2022-091979 | A | 21 June 2022 |
| | | | | JP | 6673680 | B2 | 25 March 2020 |
| | | | | US | 10576619 | B2 | 03 March 2020 |
| | | | | US | 2016-0184985 | A1 | 30 June 2016 |
| | | | | US | 2020-0070335 | A1 | 05 March 2020 |
| JP | 2017-504440 | A | 09 February 2017 | CN | 105992932 | A | 05 October 2016 |
| | | | | CN | 105992932 | B | 02 July 2019 |
| | | | | EP | 3099229 | A1 | 07 December 2016 |
| | | | | EP | 3099229 | B1 | 30 October 2019 |
| | | | | JP | 6795980 | B2 | 02 December 2020 |
| | | | | US | 11039760 | B2 | 22 June 2021 |
| | | | | US | 2017-0000384 | A1 | 05 January 2017 |
| | | | | WO | 2015-113915 | A1 | 06 August 2015 |
| JP | 2021-003605 | A | 14 January 2021 | CN | 112955751 | A | 11 June 2021 |
| | | | | JP | 6774579 | B2 | 28 October 2020 |
| | | | | US | 2021-0321906 | A1 | 21 October 2021 |
| | | | | WO | 2020-045371 | A1 | 05 March 2020 |
| KR | 10-2019-0113235 | A | 08 October 2019 | None | | | |

Form PCT/ISA/210 (patent family annex) (July 2022)